Europäisches Patentamt

European Patent Office

Office européen des brevets

(19)

(11) Numéro de publication: **0 065 442**
**A1**

(12) **DEMANDE DE BREVET EUROPEEN**

(21) Numéro de dépôt: 82400771.0

(22) Date de dépôt: 28.04.82

(51) Int. Cl.³: **A 61 M 1/03, B 01 D 13/00**

(30) Priorité: 29.04.81 FR 8108831

(43) Date de publication de la demande: 24.11.82
Bulletin 82/47

(84) Etats contractants désignés: **AT BE CH DE FR GB IT LI LU NL SE**

(71) Demandeur: **Demandoix, Philippe, 15 quai Provost, F-44640 Le Pellerin (FR)**
Demandeur: **Geffard, Serge, Le Moulin de la Boizonnière, F-44320 Arthon en Retz (FR)**
Demandeur: **Rondard, Gérard, 44, rue du clos Toreau, F-44200 Nantes (FR)**

(72) Inventeur: **Demandoix, Philippe, 15 quai Provost, F-44640 Le Pellerin (FR)**
Inventeur: **Geffard, Serge, Le Moulin de la Boizonnière, F-44320 Arthon en Retz (FR)**
Inventeur: **Rondard, Gérard, 44, rue du clos Toreau, F-44200 Nantes (FR)**

(74) Mandataire: **Phélip, Bruno et al, c/o Cabinet Harlé & Phélip 21, rue de La Rochefoucauld, F-75009 Paris (FR)**

(54) Procédé et installation de nettoyage et décolmatage d'un système à fibres creuses semi-perméables, notamment d'un rein artificiel.

(57) Une installation comprend une canalisation ($f_1$) d'introduction de liquide de lavage, notamment de l'eau traitée du réseau, pourvue de moyens ($EVR_1$) de réglage de pression et de débit, une canalisation ($g_1$) d'introduction d'un liquide de conditionnement comportant une pompe distributrice (P) actionnée par le liquide de lavage et coopérant avec un dispositif de dosage ($EVR_2$), les deux canalisation se rejoignant (en J) pour assurer le mélange des deux liquides et étant ensuite reliées à un ensemble de tuyaux d'entrée/sortie des deux compartiments de l'échangeur (S) qui sont commandés par un ensemble ($E_1$) d'électrovannes ($EV_1$, $EV_2$, $EV_3$, $EV_4$) et un dispositif (C) de commande et régulation automatique, ou manuelle, des paramètres des différents cycles de nettoyage préalable, de rétrofiltration, de stérilisation et de rinçage du compartiment intérieur et du compartiment extérieur de l'appareil échangeur (S).

Procédé et installation de nettoyage et décolmatage
d'un système à fibres creuses semi-perméables, notamment d'un rein artificiel

La présente invention concerne un procédé
et une installation de nettoyage et décolmatage d'un
système à fibres creuses semi-perméables, notamment
d'un rein artificiel.

Dans les domaines de l'ultrafiltration et
des traitements par osmose et par dialyse, on utilise
principalement des appareils qu'on peut classer en
deux catégories:

-les appareils à échangeurs à membranes
semi-perméables, et les appareils à échangeurs à fibres creuses semi-perméables.

Dans les deux catégories d'appareils, l'échangeur se compose de deux compartiments dont l'un
est parcouru par le fluide à traiter. Dans l'ultrafiltration, on recueille dans l'autre compartiment
les produits passant à travers la membrane ou les
fibres. Dans la dialyse l'autre compartiment est parcouru par un fluide de traitement. Dans tous les cas,
les deux compartiments sont séparés l'un de l'autre
par une paroi semi-perméable qui est constituée, pour
la première catégorie d'appareils, par une ou plusieurs membranes continues et, pour la seconde catégorie d'appareils, par les parois périphériques des
différentes fibres creuses.

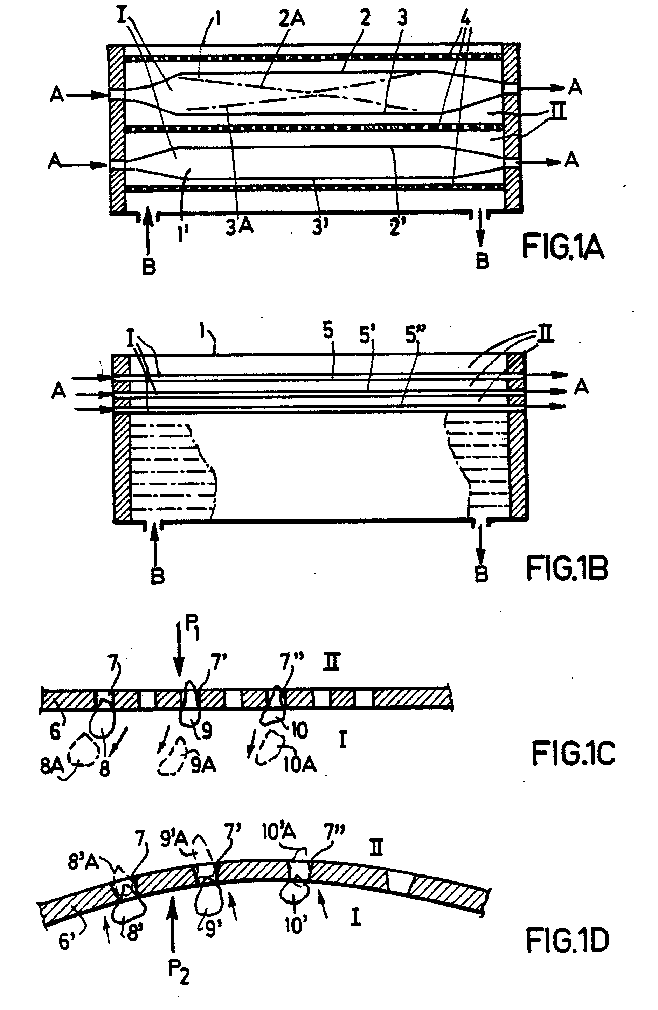

Pour bien définir la portée de l'invention
par rapport à l'art antérieur, on va d'abord donner
une brève description des deux catégories d'appareils, en référence aux figures 1A à 1D, en effectuant
la comparaison des deux structures dans le cas d'opérations de dialyse. Sur la figure 1A, on a représenté

en coupe schématique un appareil à membranes qui comporte en fait un certain nombre de pochons tels que 1, 1', délimités respectivement par deux membranes 2,3 et 2',3' et séparés les uns des autres par des grilles intercalaires 4. Dans les pochons s'écoule le fluide à traiter alors que,dans le volume extérieur aux pochons s'écoule le fluide de traitement. Les membranes étant souples,il est par conséquent possible que le volume I des pochons soit augmenté ou réduit par application d'une pression respectivement à l'intérieur/à l'extérieur des pochons. On verra dans la suite quelles sont les conséquences d'une telle application de pression.

Au contraite,dans la structure à fibres creuses semi-perméables représentée sur la figure 1B,il est prévu dans une enceinte semblable un grand nombre de fibres creuses semi-perméables telles que 5,5' 5" etc.....dont les parois périphériques constituent des éléments discontinus de membrane. Cependant,à la différence de la section de la figure 1A,les fibres creuses de la figure 1B ne se déforment pratiquement pas sous l'effet d'une augmentation de la pression intérieure ou extérieure.

Sur la figure 1C,on a illustré par une vue en coupe une partie 6 de paroi semi-perméable qui peut aussi bien représenter une partie d'une des membranes 2,3 de délimitation de pochons lorsqu'elle se trouve à l'état plan,qu'une partie de la paroi périphérique d'une fibre creuses 5,5'. Dans cette partie de paroi, on a figuré en 7, 7' et 7" des pores existant dans la paroi 6. Lorsque l'appareil est utilisé pour tout traitement osmotique,le fluide à filtrer ou à traiter A passe à l'intérieur des pochons ou des fibres creuses,comme indiqué en I,tandis que le fluide de traitement B s'écoule à l'extérieur,comme indiqué en II, et il se produit un échange entre les deux fluides. Les déchets contenus dans le fluide A passent au travers des pores 7, 7', 7" ,comme indiqué par les particules schématisées en $8_A$, $9_A$, $10_A$ sur la figure 1C. De petites particules passent facilement au travers des pores mais des particules plus grosses les obstruent et il devient par conséquent nécessaire d'effectuer un nettoyage et un décolmatage des parois semi-perméables. Une telle opération est effectuée à la fin du cycle d'utilisation de l'appareil échangeur. On a indiqué sur la figure 1D ce qui se passe lorsqu'on met en pression,comme indiqué en $P_2$,le volume intérieur I d'un pochon délimité par une membrane souple 6'; les pores 7, 7', 7" ,initialement de forme cylindrique,prennent une forme conique se rétrécissant vers l'intérieur,ce qui a tendance à gêner le passage des particules de l'intérieur vers l'extérieur. En considérant à nouveau la figure 1D, on se rend compte également que,dans le cas d'une fibre creuse cylindrique,une augmentation de la pression dans le volume intérieur I a tendance à augmenter l'effet de coincement des particules 8', 9',10' dans les pores 7, 7', 7" ,comme indiqué en 8'A, 9'A et 10'A. En conséquence,dans les deux cas,une augmentation de la pression à l'intérieur d'un pochon ou d'une fibre creuse a tendance à gêner le passage des particules de l'intérieur vers l'extérieur.

En considérant le processus inverse,c'est-à-dire une augmentation de pression à l'extérieur d'un pochon ou d'une fibre creuse,comme indiqué en P1 sur la figure 1D,il se passe les phénomènes suivants:

-pour le pochon,les deux membranes 2,3 assurant sa délimitation sont poussées l'une vers l'autre et finalement l'une contre l'autre, en empêchant alors le passage du liquide correspondant, comme le montrent les lignes en trait mixte $2_A$ et $3_A$ sur la figure 1A,.Au contraire pour la fibre creuse, l'augmentation de pression dans le volume II,figure 1C,provoque l'éjection des particules 8, 9,10 vers le volume intérieur I,comme indiqué en 8A,9A et 10A.L'étude comparative donnée ci-dessus fait bien ressortir les différences qui existent entre les systèmes à pochons et les systèmes à fibres creuses. Outre l'augmentation importante de surface active qu'on obtient avec les systèmes à fibres creuses, ceux-ci offrent,du fait de la rigidité des parois des fibres,de meilleures possibilités de nettoyage et décolmatage et sont de plus en plus utilisés dans les domaines de l'ultrafiltration et des traitements par osmose,par dialyse,etc...Le procédé et l'installation selon l'invention se rapportent essentiellement au domaine des appareils échangeurs à fibres creuses semi-perméables,notamment des reins artificiels. La présente invention sera décrite dans la suite en référence à ce domaine d'application,qui devra cependant être considéré comme n'ayant aucun effet limitatif sur la portée de l'invention,comme cela sera précisé dans la suite.

Un rein artificiel est un appareil permettant d'effectuer des traitements d'hémodialyse ayant pour but d'épurer le sang des malades n'ayant plus leurs fonctions rénales. Selon cette technique,l'épuration du sang s'effectue d'une part à l'aide d'un générateur créant un bain de dialyse d'une formule chimique identique à un sang normal et d'autre part au moyen d'un échangeur. Ce dernier,qu'il soit du type à fibres capillaires ou du type à membrane, se compose de deux compartiments dans l'un desquels circule le bain de dialyse tandis que le sang contenant un excès de déchets à éliminer circule dans l'autre compartiment,qui est séparé du premier par la ou les parois semiperméables définies ci-dessus. L'échange entre le bain de dialyse et le sang à traiter s'effectue en maintenant dans le compartiment-bain une pression inférieure à celle du sang à traiter afin de permettre une diffusion entre les deux solutions de concentrations différentes,le trajet s'établissant de la solution la plus concentrée vers la solution la moins concentrée. Après chaque utilisation de l'échangeur,on doit procéder aux opérations de nettoyage et de stérilisation pour permettre son réemploi. Les procédés actuellement utilisés consistent dans le passage et la montée en pression d'une solution de nettoyage dans le circuit sang,puis dans l'exécution d'une opération de stérilisation à l'aide d'un liquide approprié dans le circuit-sang puis dans le circuit-bain.

Ainsi,dans le brevet US N° 4.209.402, on décrit un procédé et un appareil de nettoyage de rein artificiel,où on utilise un système de lavage dynamique à tuyau souple de sortie qui est expansible et contractable pour créer dans le compartiment considéré des variations de pression servant à déloger les déchets obturant les pores. Il apparaît alors qu'on rencontre les inconvénients définis ci-dessus en ce qui concerne la mise en pression du volume I, c'est-à-dire le côté-sang,car l'expansion de la membrane a au contraire tendance à coincer davantage les particules retenues dans les pores. En outre,bien que la description de ce brevet US 4.209.402 indique que la configuration de la membrane n'est pas critique pour l'invention considérée,on ne fait absolument pas mention d'un rein artificiel à fibres creuses.

D'après le brevet US 3.920.030,on connaît un dispositif de nettoyage et de stérilisation de reins artificiels,pour lequel on ne fait également aucune mention de son application à un rein artificiel à fibres creuses semiperméables ,. Conformément à ce brevet,on opère également en produisant dans le compartiment-sang une rapide impulsion de pression pour provoquer une expansion de la membrane du rein artificiel en vue de déloger les particules obstruant les pores. On rencontre évidemment les inconvénients mentionnés ci-dessus; en outre il est à noter qu'on opère avec une pompe électrique de dosage qui produit des impulsions périodiques de fluide à l'aide d'une roue à galets écrasant un tuyau souple.

L'invention a pour but de remédier aux inconvénients des réalisations connues à l'aide d'un procédé et d'une installation permettant un nettoyage poussé des compartiments d'un système échangeur,un décolmatage parfait des parois des fibres creuses semiperméables,ainsi que, si nécessaire,d'une stérilisation efficace des compartiments; de plus, le déroulement des opérations,notamment le dosage des constituants du mélange de traitement,s'effectue automatiquement.

L'invention a pour objet un procédé pour le nettoyage et le décolmatage d'un système à fibres creuses semi-perméables,lequel système présente un volume intérieur,défini par les cavités des fibres creuses et balayé par le fluide à traiter,et un volume extérieur aux fibres,ledit procédé étant caractérisé en ce que,après un cycle de traitement,on opère au moins un cycle de rétrofiltration dans lequel,après avoir interrompu la circulation du fluide à traiter à l'intérieur des fibres creuses,on met en pression le volume extérieur à celles-ci à l'aide d'un liquide de lavage,pour assurer ainsi un nettoyage des pores des fibres de l'extérieur vers l'intérieur, le liquide de lavage,ayant traversé les pores en entraînant les déchets coincés dans ceux-ci,étant évacué.

Dans son application à la dialyse,le procédé de l'invention implique également l'interruption de la circulation du liquide de traitement dans le volume extérieur aux fibres et son remplacement par le liquide de nettoyage en vue de la rétrofiltration. Ainsi, d'une façon générale , le procédé de l'invention se caractérise en ce que,après une étape de traitement proprement dit, il comprend au moins une étape de rétrofiltration.

Selon l'invention l'étape de rétrofiltration avec un liquide de lavage peut être avantageusement précédée d'une étape de prélavage en soi connue ayant pour but d'éliminer les déchets les plus grossiers présents à l'extérieur des fibres. En fait un tel prélavage est très utile dans les opérations de type industriels.

Selon l'invention,l'étape de rétrofiltration peut être suivie d'au moins une stérilisation des fibres. Celle-ci est obligatoire pour certaines applications,notamment pour le rein artificiel.

Dans un mode particulier de réalisation utilisable en dialyse,le procédé de nettoyage et décolmatage selon l'invention d'un système à fibres creuses semi-perméables ,notamment d'un rein artificiel,se composant d'un volume intérieur,défini par les cavités des fibres creuses et balayé par le liquide à traiter,et d'un volume extérieur aux fibres, balayé par le liquide de conditionnement est caractérisé en ce qu'il comprend entre un cycle de prénettoyage et au moins un cycle de stérilisation, un cycle de rétrofiltration dans lequel on met en pression le volume extérieur aux fibres creuses à l'aide d'un liquide de lavage, notamment de l'eau traitée du réseau,pour assurer ainsi un nettoyage des pores des fibres de l'extérieur vers l'intérieur,le liquide de lavage,ayant traversé les pores et entraînant les déchets,étant ensuite évacué.

Selon d'autres particularités du procédé conforme à l'invention:

-dans le cycle de rétrofiltration,on assure la mise en pression du volume extérieur aux fibres creuses , en fermant le circuit contenant ledit volume extérieur, en modulant le degré d'ouverture de la vanne d'entrée de ce circuit pour régler la pression du liquide de lavage et en maintenant ouverte la vanne de sortie du circuit contenant le volume intérieur des fibres creuses;

-dans le cas d'un rein artificiel se composant d'un compartiment-sang et d'un compartiment-bain de dialyse,le procédé comprend la succession de cycles suivants après utilisation du rein artificiel:

a) un cycle de nettoyage préalable du compartiment-sang en opérant sans pression,

b)un cycle de rétrofiltration où le compartiment-bain est mis en pression avec un liquide de lavage,notamment de l'eau traitée,pour le nettoyage des pores des fibres creuses;

c)un cycle de stérilisation du compartiment-sang à l'aide d'un mélange d'eau traitée et d'un agent de conditionnement,notamment du formol,dosé de façon à obtenir en permanence le degré de dilution correct dans le mélange,

d)un cycle de stérilisation du compartiment-bain à l'aide du liquide de conditionnement,

-complémentairement,après la succession de cycles sus-mentionnée,on effectue,avant le réemploi du rein artificiel,un rinçage du compartiment-bain à l'aide d'un liquide approprié,notamment de l'eau traitée,et un rinçage du compartiment-sang à l'aide de sérum physiologique.

Le dosage du liquide de conditionnement dans le mélange de stérilisation est effectué en faisant intervenir trois moyens:

a) prélèvement d'un volume initial de liquide qui soit d'une part constant et d'autre part suffisant pour l'éxécution d'un cycle de stérilisation complet,

b)maintien de la pression de refoulement constante pendant distribution du liquide pendant la durée du cycle, et

c)captage du degré de dilution du mélange produit et correction correspondante de ce degré de dilution en cours d'opération par réglage du débit.

L'invention concerne également une installation pour la mise en oeuvre du procédé défini ci-dessus,cette installation étant caractérisée en ce qu'elle comprend:

-une canalisation d'introduction de liquide de lavage,notamment de l'eau traitée du réseau,pourvue de moyens de réglage de pression et de débit,

-une canalisation d'introduction d'un liquide de conditionnement,comportant une pompe distributrice actionnée par le liquide de lavage et coopérant avec un dispositif de dosage,

-les deux canalisations se rejoignant pour assurer le mélange des deux liquides et étant ensuite reliées à un ensemble de tuyaux d'entrée/sortie des deux compartiments de l'échangeur qui sont commandés par des électrovannes, et

-un dispositif de commande et régulation automatique,ou manuelle,des paramètres des différents cycles de nettoyage préalable, de rétrofiltration, de stérilisation et de rinçage des compartiments intérieur et extérieur de l'appareil échangeur.

Selon l'autres particularités de l'installation conforme à l'invention:

-la pompe distributrice de liquide de conditionnement est constituée par un cylindre divisé par une cloison médiane en deux compartiments dans chacun desquels se déplace un piston,à savoir un piston d'actionnement,entraîné par le liquide de lavage sous la commande d'un régulateur de pression et de deux électrovannes faisant en sorte qu'une pression réglable soit exercée dans les deux courses de ce piston,et en outre un piston de pompage-refoulement se déplaçant dans l'autre compartiment dont un orifice de sortie est relié d'une part,par l'intermédiaire d'un premier clapet anti-retour,à un réservoir de liquide de conditionnement et d'autre part,par l'intermédiaire d'un second clapet anti-retour,avec un dispositif de dosage du liquide de conditionnement refoulé par ledit piston.

Le dispositif de dosage de liquide de conditionnement comprend une vanne du type à aiguille ou à pointeau qui est branchée en aval du moyen de pompage avant le point de jonction avec la canalisation d'introduction de liquide de lavage.

Le dispositif de dosage de liquide de conditionnement comprend un organe de régulation de débit branché entre le moyen de pompage et le point de jonction avec la canalisation d'introduction de liquide de lavage,ledit organe étant commandé par un capteur de dilution qui est branché après le point de jonction précité dans la canalisation de passage du mélange de traitement.

D'autres avantages et caractéristiques de l'invention seront mis en évidence dans la suite de la description,donnée à titre d'exemple non limitatif,en référence aux dessins annexés dans lesquels:

Fig.1A représente en coupe schématique très simplifiée un appareil échangeur à membranes semi-perméables,

Fig.1B représente également de façon très schématique un appareil échangeur à fibres creuses semi-perméables;

Figs.1C et 1D sont des vues en coupe d'une paroi semi-perméable permettant d'expliquer les phénomènes qui se passent dans les deux types d'échangeurs précités;

Fig.2 est un schéma synoptique permettant d'expliquer les particularités essentielles du procédé et de l'installation selon l'invention;

Figs.3, 4, 5, 6,7 représentent un exemple d'application de l'installation selon l'invention au nettoyage et décolmatage d'un rein artificiel;

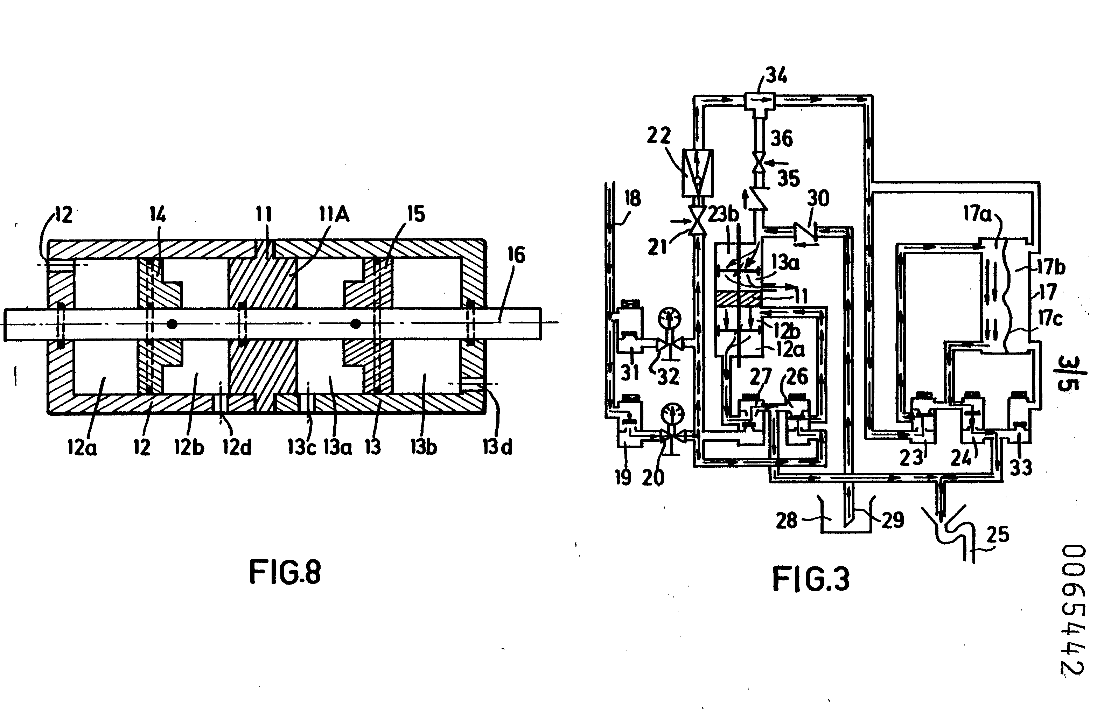

Fig.3 montrant le cycle de rinçage du compartiment-sang;

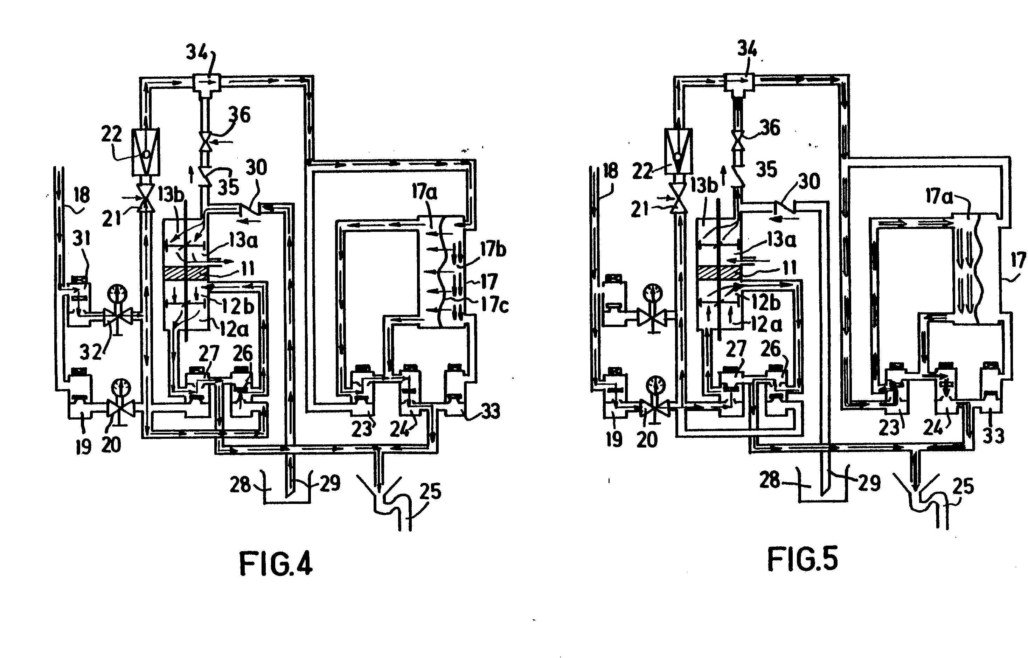

Fig.4 montrant le cycle de rétrofiltration;

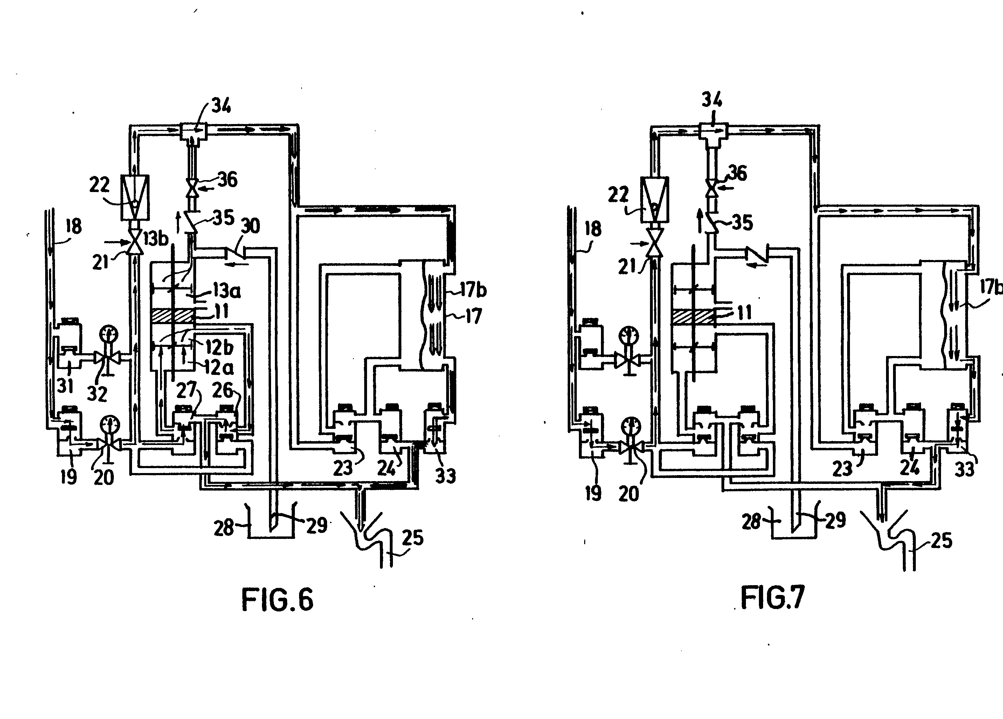

Fig.5 montrant le cycle de stérilisation du circuit-sang;

Fig.6 montrant le cycle de stérilisation du circuit-bain;

Fig.7 montrant l'étape de rinçage avant dialyse;

Fig.8 représentant une vue en coupe longitudinale du dispositif de pompage de liquide de traitement, notamment du formol.

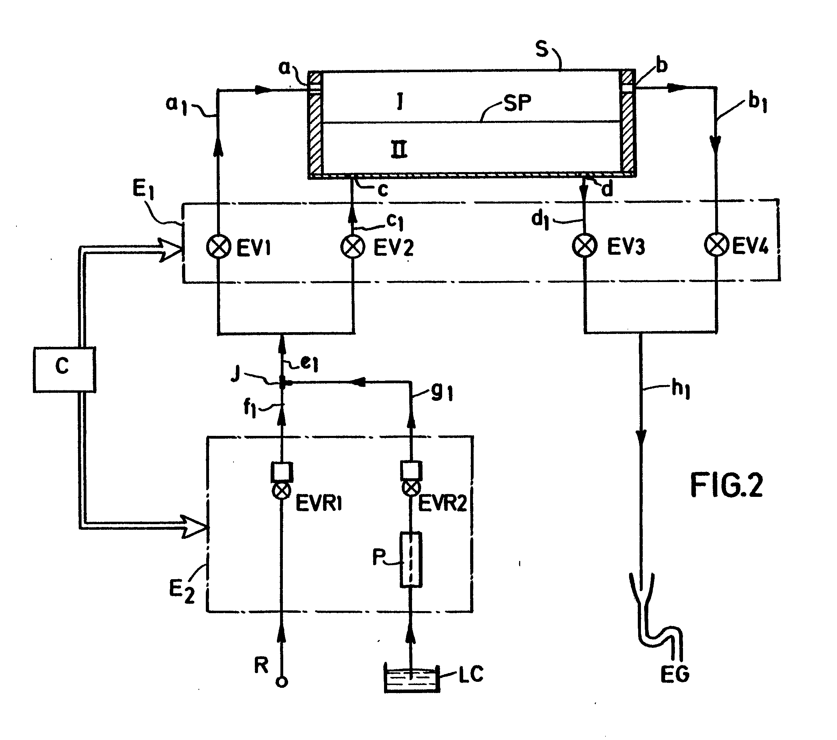

Comme cela a été précisé ci-dessus,le procédé et l'installation selon l'invention sont applicables essentiellement au domaine des systèmes à fibres creuses semi-perméables. Un tel système a été désigné sur la figure 2 par S et il comporte un carter dans lequel on a schématisé les deux compartiments I et II séparés l'un de l'autre par une paroi semi-perméable SP. Comme cela a été expliqué ci-dessus en référence aux figures 1A à 1D, le compartiment I représente le volume intérieur des fibres creuses dans lesquelles passe le liquide à traiter, à savoir le sang dans le cas d'un rein artificiel; tandis que le volume II correspond au volume extérieur aux fibres. Dans une dialyse, le volume II est parcouru par le liquide de traitement, à savoir le bain de dialyse dans le cas d'un rein artificiel. Comme on l'a précisé ci-dessus, c'est d'ailleurs en référence à une telle application que l'invention va être décrite dans la suite de façon à mettre en évidence concrètement ses caractéristiques essentielles.

Après chaque utilisation d'un rein artificiel, on doit procéder aux opérations de nettoyage et de stérilisation de l'appareil pour permettre son réemploi. Pour remplir cette fonction, le procédé selon l'invention comprend des étapes successives, savoir le nettoyage et la stérilisation après dialyse puis le rinçage avant réemploi. La succession des étapes est la suivante:

a) un cycle de pré-nettoyage dans lequel on effectue une élimination grossière des déchets en suspension dans les fibres creuses (ou capillaires) en faisant passer, dans le compartiment-sang, de l'eau du réseau, préalablement traitée et qui est ensuite rejetée à l'égout,

b) un cycle de rétrofiltration dans lequel intervient également de l'eau traitée du réseau qu'on introduit dans le compartiment-bain, dont la sortie a été au préalable fermée,afin d'obliger cette eau à passer au travers des parois semi-perméables des fibres creuses de l'extérieur vers l'intérieur pour produire un décolmatage de ces fibres,

c)un cycle de stérilisation du compartiment-sang dans lequel on utilise un mélange d'eau traitée du réseau et d'un liquide de conditionnement,notamment du formol,qu'on fait passer dans le compartiment-sang de l'appareil échangeur, et

d)un cycle de stérilisation du compartiment-bain,dans lequel on opère de la même manière que pour la stérilisation du compartiment-sang.

On procède ensuite à l'étape de rinçage avant réemploi,où l'on utilise pour le compartiment-bain de l'eau traitée du réseau et pour le compartiment-sang un liquide de conditionnement tel qu'un sérum physiologique.

La durée des cycles, les pressions,les débits et les dosages,qui sont modulables,par exemple en fonction des prescriptions du médecin,sont réglés à l'aide d'une commande automatique qui peut être de nature électronique,électromécanique ou autres.

La figure 2 montre schématiquement comment s'effectue la mise en oeuvre du procédé selon l'invention. L'eau du réseau,préalablement traitée,arrive en R et elle passe dans un ensemble EVR1 de commande de débit et de régulation de pression pour parvenir ensuite à une jonction J. Cette eau peut parvenir ensuite par l'intermédiaire des deux tuyaux de dérivation $a_1$, $c_1$, respectivement à l'entrée $a$ du compartiment I et à l'entrée $c$ du compartiment II,les deux dérivations étant commandées par des électrovannes EV1 et EV2. Pour produire le mélange de stérilisation qui est nécessaire dans certaines opérations,on fait arriver à la jonction J,par l'intermédiaire du tuyau $g_1$, du liquide de conditionnement,notamment du formol,qui est aspiré à partir d'un réservoir schématisé en LC à l'aide d'une pompe distributrice schématisée en P et coopérant avec un moyen de dosage et régulation de pression désigné par EVR2. L'eau traitée ou le mélange de stérilisation sont évacués des compartiments respectifs par l'intermédiaire de l'orifice de sortie $\underline{b}$ et du tuyau $b_1$, commandé par l'électrovanne EV4, pour le compartiment I,et par l'intermédiaire de l'orifice de sortie $\underline{d}$ et du tuyau $d_1$, commandé par l'électrovanne EV3,pour le compartiment II,la canalisation de décharge à l'égout EG étant désignée par $h_1$. Sur la figure 2,on a désigné par $E_1$ l'ensemble formé par les électrovannes EV1, EV2, EV3 EV4 de commande des différents tuyaux d'entrée et de sortie des compartiments I et II, et par $E_2$ l'ensemble de distribution de fluides,ces deux ensembles étant commandés par le dispositif schématisé en C.

L'établissement des différents circuits permettant l'exécution des divers cycles du procédé ressort parfaitement d'un examen de la figure 2,où des flèches indiquent les sens d'écoulement des différents fluides. Ainsi par exemple le cycle de rétrofiltration,qui correspond à la caractéristique essentielle de l'invention,est effectué de la manière suivante: on ouvre EVR1 pour laisser passer l'eau traitée du réseau et pour assurer sa régulation de pression,on ferme EVR2 car on n'a pas besoin du liquide de conditionnement,on ferme l'électrovanne EV1 pour empêcher l'eau de pénétrer dans le compartiment 1 , on ouvre l'électrovanne EV2 pour permettre à l'eau de pénétrer dans le compartiment 2,on ferme l'électrovanne EV3 pour interdire la sortie de l'eau par cette voie et pour l'obliger à traverser la paroi semi-perméable SP et on ouvre l'électrovanne EV4 pour évacuer l'eau contenant les déchets jusqu'à l'égout EG.

On va maintenant décrire l'installation de nettoyage et décolmatage de système à fibres creuses semi-perméables en référence à son application à un rein artificiel, en considérant les figures 3 à 7, qui sont des schémas de l'installation montrant l'établissement des différents circuits permettant l'exécution des différentes étapes du processus. Le rein artificiel a été désigné par 17 et il est divisé en un compartiment-sang 17a et un compartiment-bain 17b par une paroi semi-perméable 17c, qui représente en fait toutes les parois périphériques des fibres creuses semi-perméables intervenant dans l'échangeur. L'eau traitée du réseau arrive en 18, le récipient de liquide de stérilisation est indiqué en 28 et l'égout en 25. La commande de l'arrivée d'eau est assurée par une électrovanne 19, la régulation de pression est assurée par un régulateur 20 et en outre le débit d'eau est réglé au moyen d'un organe 11, par exemple une vanne à pointeau 21; le contrôle de ce débit est assuré à l'aide d'un débit-mètre de façade 22, l'eau parvenant ensuite au raccord en T 34 où, dans certaines opérations du processus, elle doit recevoir une dose de liquide de conditionnement pour que le mélange ainsi formé remplisse sa fonction de stérilisation.

On va maintenant décrire le système de distribution et de dosage qui permet d'injecter au point 34 la dose appropriée de liquide de conditionnement. Le problème consiste à faire passer dans les compartiments 17a et 17b un mélange de stérilisation dans lequel le liquide de conditionnement, notamment du formol, est dilué à un degré déterminé. Pour obtenir la meilleure efficacité en ce qui concerne le maintien correct du degré de dilution, la réduction des frais de fabrication et une rentabilité d'exploitation optimale de l'appareil, les inventeurs ont eu l'idée d'utiliser comme distributeur une pompe à double piston coulissant dans un cylindre divisé en deux chambres, le premier piston, remplissant la fonction d'actionnement, étant entraîné par l'eau du réseau tandis que le second piston, remplissant la fonction de pompage et de refoulement du formol, coopère avec un dispositif de dosage pour injecter le formol avec le degré de dilution approprié dans l'eau parvenant au raccord 34.

On a représenté sur la figure 1, la pompe distributrice 1 qui est constituée d'un corps cylindrique divisé par une cloison médiane 11A dans deux compartiments égaux 12 et 13 dans chacun desquels se déplace un piston respectif 14, 15, les deux pistons étant solidaires d'une même tige 16 qui peut coulisser au travers des fonds du corps et de la cloison médiane. Le piston 14 partage le compartiment 12 en deux chambres 12a et 12b; de même le piston 15 partage le compartiment 13 en deux chambres 13a et 13b, lesdites chambres ayant des volumes qui varient en fonction du déplacement des pistons. Chacune desdites chambres comporte un orifice de mise en communication avec le réseau de canalisations reliant la pompe 11 et l'échangeur 17 aux sources d'alimenta- ion en eau traitée ou en formol, au rejet à l'égout ou bien à l'atmosphère. Ainsi :

- la chambre 12a comporte un orifice 12c d'alimentation en eau traitée ou d'évacuation d'air ou d'eau à l'égout,

- la chambre 12b comporte un orifice 12d d'alimentation en eau traitée ou d'évacuation d'air ou d'eau à l'égout,

- la chambre 13a comporte un orifice 13c de mise à l'air, et

- la chambre 13b comporte un orifice 13d d'aspiration et de refoulement de formol. Pour assurer la commande du piston d'actionnement 14 de la pompe distributrice, il est prévu deux électrovannes à trois voies 26, 27. Le compartiment de pompage 13 comprend une chambre de pompage-refoulement de formol 13b et une chambre de mise à l'air 13a. Quand le piston d'actionnement 14 est déplacé vers la gauche, en considérant la figure 8, le piston 15 aspire du formol se trouvant dans le réservoir 28, notamment son emballage d'origine, par l'intermédiaire d'un tuyau 29 et d'un clapet anti-retour 30. Ensuite, lors de l'inversion de la course du piston d'actionnement, le formol contenu dans la chambre 13b est refoulé en direction du raccord 34 sous une pression constante exercée par l'intermédiaire du piston d'actionnement 14 et réglée automatiquement à l'aide du régulateur 20. Pour obtenir un degré de dilution du formol dans l'eau qui soit correct et constant pendant les différents cycles de stérilisation, les inventeurs font intervenir en combinaison les trois moyens suivants :

a) cylindrée de la chambre 13b de valeur suffisante pour assurer la distribution du formol pendant toute la durée d'un cycle de stérilisation,

b) pression constante de refoulement du formol,

c) coopération avec un moyen de dosage constitué par une vanne à pointeau ou à aiguille, comme indiqué en 36, ou bien, pour augmenter la précision, coopération de cette vanne avec un capteur de dilution de mélange branché en aval du raccord 34 et réagissant sur la vanne à aiguille pour modifier son réglage.

A la sortie du raccord 34, l'eau traitée ou le mélange de stérilisation parvient à l'appareil échangeur 17 par l'intermédiaire de canalisations qui sont commandées à l'aide des électrovannes 23, 24 et 33. A cet égard il est d'ailleurs à noter que, par comparaison à la structure de principe de la figure 2, l'incorporation de l'électrovanne à trois voies 23 permet d'une part de supprimer une électrovanne et d'autre part, pendant la phase de rétrofiltration, de faire sortir l'eau de décolmatage ayant traversé la membrane semiperméable 17c à la fois par l'orifice d'entrée et l'orifice de sortie du compartiment-sang 17a, le débit étant alors assez faible et l'eau de décolmatage contenant les déchets étant mieux évacuée.

On va maintenant décrire le fonctionnement de l'installation selon l'invention pendant un processus complet d'utilisation comprenant l'étape de nettoyage et stérilisation après utilisation et l'étape de rinçage avant réemploi.

Comme indiqué sur la figure 3, correspondant au cycle de rinçage du compartiment-sang 17a de l'échangeur 17, dont le but est d'assurer une première élimination de la majeure partie des déchets en suspension dans les capillaires, le compartiment 17a est mis en communication avec la canalisation d'eau traitée 18 par ouverture de l'électrovanne 19; la pression d'arrivée d'eau est régulée à l'aide du régulateur de pression 20, le débit est limité au moyen de la vanne à pointeau 21 et l'admission de l'eau traitée dans le compartiment-sang 17a s'effectue par ouverture de l'électrovanne à trois voies 23; après passage dans ce compartiment, l'eau chargée de déchets est évacuée par l'intermédiaire de l'électrovanne 24 pour être rejetée à l'égout en 25. Simultanément l'eau traitée est également admise dans la chambre 12b de la pompe distributrice 11 par l'intermédiaire d'une électrovanne à trois voies 26, ce qui provoque le déplacement du piston 14, lequel expulse par l'orifice 12c l'air ou l'eau contenu dans la chambre 12a vers l'égout 25 par l'intermédiaire d'une électrovanne à trois voies 27. La translation du piston 14 provoque l'entraînement du piston 15; l'air contenu dans la chambre 13a est expulsé à l'extérieur par l'orifice 13c et le formol est alors aspiré à partir du réservoir 28 dans la chambre 13b qui est remplie jusqu'en fin de course du piston 15.

L'installation est maintenant prête à effectuer l'opération de rétrofiltration, mise en évidence sur la figure 4. On assure alors la mise en pression du compartiment-bain 17b de l'échangeur 17 par admission d'eau traitée provenant de la canalisation 8 par ouverture d'une électrovanne 31 en aval de laquelle est branché un régulateur de pression 32, le débit étant fonction de ladite pression et de la perméabilité de la paroi semiperméable 17c de l'échangeur 17. L'électrovanne 33 branchée à la sortie du compartiment-bain est fermée de manière que celui-ci monte en pression; l'eau cherchant à traverser les capillaires de la paroi semiperméable 17c provoque un décolmatage de celle-ci. Le liquide contenant les dépôts décollés est évacué par les deux orifices situés aux extrémités du compartiment-sang 17a pour gagner le point de rejet à l'égout 25 par l'intermédiaire des électrovannes 23 et 24. Il est à noter qu'il est prévu l'ensemble électrovanne-régulateur de pression 31, 32, qui semble intervenir de façon superflue, pour permettre une commande manuelle de l'opération de rétrofiltration car celle-ci ne fait intervenir qu'une régulation de pression du fait que l'eau ne peut passer qu'au travers de la paroi semiperméable.

Sur la figure 5, on a mis en évidence le cycle de stérilisation du circuit-sang. Après ouverture de l'électrovanne 19, l'eau traitée dont la pression est réglée par le régulateur 20 et dont le débit est limité par la vanne-pointeau 21, parvient au raccord mélangeur 34. L'eau traitée est également admise dans la chambre 12a de la pompe distributrice par l'intermédiaire de l'électrovanne à trois voies 27, en chassant le piston 14 du compartiment 12, ce qui provoque le rejet à l'égout 25, à travers l'électrovanne à trois voies 26, de l'air ou l'eau contenu dans la chambre 12b. Simultanément de l'air pénètre dans la chambre 13a du compartiment 13 et le formol contenu dans la chambre 13b est refoulé vers le raccort mélangeur 34 au travers d'un clapet anti-retour 35 et du moyen de dosage précité 36. Le mélange eau-formol est canalisé par l'intermédiaire de l'électrovanne à trois voies 23 vers le compartiment-sang 17a qu'il traverse pour être ensuite évacué à l'égout 25 au travers de l'électrovanne 24; lorsque ce circuit est complètement établi, cette électrovanne est fermée pour maintenir étanche le circuit-sang pendant la période où le mélange stérilisant doit exercer son action.

La figure 6 met en évidence le cycle de stérilisation du circuit-bain, à la suite de celle du circuit-sang. Après fermeture des électrovannes 23 et 24 et après ouverture de l'électrovanne 33, le mélange stérilisant passe dans le compartiment bain 17b de l'échangeur 17 pour être ensuite évacué à l'égout 25 à travers l'électrovanne 33. Quand le circuit est complètement établi, on procède à la fermeture de cette électrovanne pour permettre au mélange stérilisant d'exercer son action dans le circuit-bain.

Après exécution des différentes opérations correspondant à l'étape de nettoyage et de stérilisation, on procède alors à l'exécution de l'étape de rinçage avant réemploi, comme indiqué sur la figure 7, cette opération consistant à évacuer le mélange stérilisant contenu dans le circuit-bain et à effectuer un rinçage à l'eau qui est évacué à l'égout 25 par l'intermédiaire de l'électrovanne 33. Le circuit-sang peut être rincé indépendamment par l'utilisateur à l'aide d'un sérum physiologique.

Les durées des cycles, les pressions, les débits et les dosages sont modulables en fonction des prescriptions du médecin. Le pilotage de l'installation peut être réalisé à l'aide d'une commande automatique de nature électronique, électromécanique ou autres. Egalement, il est à noter qu'on peut assurer une surveillance électronique du dosage de formol coopérant avec un système d'alarme.

Bien entendu l'invention n'est pas limitée aux exemples de réalisation ci-dessus décrits et représentés, à partir desquels on pourra prévoir d'autres modes et d'autres formes de réalisations sans sortir pour cela du cadre de l'invention.Ainsi l'invention est applicable à tous les do-

0065442

maines où interviennent des systèmes à fibres creuses semiperméables, en particulier les domaines suivants : dessalement d'eau de mer, ultrafiltration de lacto-sérum ou autres produits laitiers, tels que le lait, hémodialyse, plasmaphorèse, hémofiltration, traitements osmotiques, dans les domaines industriels les plus divers : biologie, produits alimentaires, et autres.

## REVENDICATIONS

1. Procédé pour le nettoyage et le décolmatage d'un système à fibres creuses semi-perméables, lequel système présente un volume intérieur, défini par les cavités des fibres creuses et balayé par le fluide à traiter, et un volume extérieur aux fibres, ledit procédé étant caractérisé en ce que, après un cycle de traitement, on opère au moins un cycle de rétrofiltration dans lequel, après avoir interrompu la circulation du fluide à traiter à l'intérieur des fibres creuses, on met en pression le volume extérieur à celles-ci à l'aide d'un liquide de lavage, pour assurer ainsi un nettoyage des pores des fibres, de l'extérieur vers l'intérieur, le liquide de lavage ayant traversé les pores en entraînant les déchets coincés dans ceux-ci, étant évacué.

2. Procédé selon la revendication 1, applicable aux opérations de dialyse/en ce qu'-caractérisé il implique également l'interruption de la circulation du liquide de traitement dans le volume extérieur aux fibres et son remplacement par le liquide de nettoyage en vue de la rétrofiltration.

3. Procédé selon l'une des revendications 1 ou 2, caractérisé en ce que l'étape de rétrofiltration est précédée d'une étape de prélavage en soi connue ayant pour but d'éliminer les déchets les plus grossiers présents à l'extérieur des fibres.

4. Procédé selon l'une quelconque des revendications 1 à 3, caractérisé en ce que l'étape de rétrofiltration est suivie d'une stérilisation des fibres.

5. Procédé selon l'une quelconque des revendications 1 à 4 applicable à un système de dialyse, notamment à un rein artificiel, se composant d'un volume intérieur, défini par les cavités des fibres creuses et balayé par le liquide à traiter, et d'un volume extérieur aux fibres, balayé par le liquide de conditionnement, comportant deux étapes essentielles, à savoir un nettoyage et une stérilisation après utilisation du système et un rinçage avant son réemploi, procédé caractérisé en ce qu'il est prévu entre un cycle de prénettoyage et le ou les cycles de stérilisation, un cycle de rétrofiltration dans lequel on met en pression le volume extérieur aux fibres creuses à l'aide d'un liquide de lavage, notamment de l'eau traitée du réseau, pour assurer ainsi un nettoyage des pores des fibres de l'extérieur vers l'intérieur, le liquide de lavage, ayant traversé les pores et entraînant les déchets, étant ensuite évacué.

6. Procédé selon l'une quelconque des revendications 1 à 5, caractérisé en ce que dans le cycle de rétrofiltration, on assure la mise en pression du volume extérieur aux fibres creuses en fermant le circuit contenant ledit volume extérieur, en réglant la pression du liquide de lavage dans ledit circuit et en maintenant ouvert le circuit contenant le volume intérieur des fibres creuses.

7. Procédé selon l'une des revendications 5 ou 6, caractérisé en ce que dans le cas d'un rein artificiel se composant d'un compartiment-sang et d'un compartiment-bain de dialyse, on réalise les cycles suivants :

a) un cycle de nettoyage préalable du compartiment-sang en opérant sans pression,

b) un cycle de rétrofiltration où le compartiment-bain est mis en pression avec un liquide de lavage, notamment de l'eau traitée, pour le nettoyage des pores des fibres creuses,

c) un cycle de stérilisation du compartiment-sang à l'aide d'un mélange d'eau traitée et d'un agent de conditionnement, notamment du formol, dosé de façon à obtenir en permanence le degré de dilution correct dans le mélange,

d) un cycle de stérilisation du compartiment-bain avec le même liquide de conditionnement.

8. Procédé selon l'une quelconque des revendications 5 à 7, caractérisé en ce que, avant le réemploi du rein artificiel, on effectue un rinçage du compartiment-bain à l'aide d'un liquide approprié, notamment de l'eau traitée, et un rinçage du compartiment-sang à l'aide de sérum physiologique, le dosage du liquide de conditionnement dans le mélange de stérilisation étant effectué en faisant intervenir trois moyens :

a) prélèvement d'un volume initial de liquide qui soit d'une part constant et d'autre part suffisant pour l'exécution d'un cycle de stérilisation complet,

b) maintien de la pression de refoulement constante pendant distribution du liquide pendant la durée du cycle, et

c) captage du degré de dilution du mélange produit et correction correspondante de ce degré de dilution en cours d'opération par réglage du débit.

9. Installation pour la mise en oeuvre du procédé selon l'une quelconque des revendications 5 à 8, caractérisée en ce qu'elle comprend :

- une canalisation ($b_1$;8) d'introduction de liquide de lavage, notamment de l'eau traitée du réseau, pourvue de moyens de réglage de pression et de débit ($EVR_1$),

- une canalisation ($g_1$;29) d'introduction d'un liquide de conditionnement comportant une pompe distributrice (P;11) actionnée par le liquide de lavage et coopérant avec un dispositif de dosage ($EVR_2$; 36);

- les deux canalisations se rejoignant (J,34) pour assurer le mélange des deux liquides et étant ensuite reliées à un ensemble de tuyaux d'entrée/sortie ($a_1$, $c_1$, $b_1$, $d_1$) des deux compartiments de l'échangeur qui sont commandés par des électrovannes ($EV_1$, $EV_2$, $EV_3$, $EV_4$; 23,24, 33)

- et un dispositif (c) de commande et régulation automatique, ou manuelle, des paramètres des différents cycles de nettoyage préalable, de rétrofiltration, de stérilisation et de rinçage des compartiments intérieur et extérieur (I, II) de l'appareil échangeur (S).

10. Installation selon la revendication 9, caractérisée en ce que la pompe distributrice de liquide de conditionnement est constituée par un cylindre (11) divisé par une cloison médiane (11A) en deux compartiments (12, 13) dans chacun desquels se déplace un piston, à savoir un piston d'actionnement (14), entraîné par le liquide de lavage sous la commande d'un régulateur de pression (20) et de deux électrovannes (26,27) faisant en sorte qu'une pression réglable soit exercée dans les deux courses de ce piston, et en outre un piston de pompage-refoulement (15) se déplaçant dans l'autre compartiment dont un orifice de sortie (13d) est re-

0065442

lié d'une part par l'intermédiaire d'un premier clapet anti-retour (30) à un réservoir (28) de liquide de conditionnement et d'autre part, par l'intermédiaire d'un second clapet anti-retour (35) à un dispositif (36) de dosage du liquide de conditionnement refoulé par ledit piston.

11. Installation selon l'une des revendications 9 ou 10, caractérisée en ce que le dispositif de dosage de liquide de conditionnement comprend une vanne du type à aiguille ou à pointeau (36) qui est branchée en aval du moyen de pompage avant le point de jonction avec la canalisation d'introduction de liquide de lavage.

12. Installation selon l'une quelconque des revendications 9 à 11, caractérisée en ce que le dispositif de dosage de liquide de conditionnement comprend un organe de régulation de débit ($EVR_2$) branché entre le moyen de pompage (P) et le point de jonction (J) avec la canalisation ($b_1$) d'introduction de liquide de lavage, ledit organe étant commandé par un capteur de dilution qui est branché après le point de jonction précité dans la canalisation ($e_1$) de passage du mélange de conditionnement.

0065442

1/5

FIG.1A

FIG.1B

FIG.1C

FIG.1D

FIG.2

0065442

FIG.3

FIG.8

FIG.4

FIG.5

FIG.6

FIG.7

0065442

Office européen
des brevets

**RAPPORT DE RECHERCHE EUROPEENNE**

Numéro de la demande

EP  82 40 0771

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| Y | FR-A-2 310 136 (BERNAS MEDICAL) * En entier; en particulier figures 1,2; page 3, lignes 19-21; page 4, lignes 4-29; page 5, lignes 2-8 * | 1-6 | A 61 M    1/03 B 01 D   13/00 |
| A | | 7,8 | |
| | --- | | |
| Y | US-A-4 166 031 (HARDY) * En entier; en particulier colonne 7, ligne 66 - colonne 8, ligne 2 * | 1-5 | |
| | --- | | |
| Y | US-A-3 786 924 (DELRO) * En entier; en particulier la figure; colonne 3, lignes 17-27, 48-50 * | 6 | |
| | --- | | |
| D,A | US-A-4 209 402 (GENTLES) * En entier; en particulier colonne 6, lignes 53-54; colonne 4, ligne 60; figures 1-3; colonne 6, ligne 44 - colonne 7, ligne 37 * | 7,9 | DOMAINES TECHNIQUES RECHERCHES (Int. Cl. ³)  A 61 M B 01 D |
| | --- | | |
| A | DE-A-2 825 134 (CORDIS DOW) * Page 23, lignes 1-2, ligne 15 - page 24, ligne 4 * | 8 | |
| | --- | | |
| A | US-A-4 096 059 (PINKERTON) * En entier * | 8,11 | |
| | --- | | |
| | -/- | | |

Le présent rapport de recherche a été établi pour toutes les revendications

| Lieu de la recherche | Date d'achèvement de la recherche | Examinateur |
|---|---|---|
| LA HAYE | 12-08-1982 | PETZUCH M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82

Office européen
des brevets

**RAPPORT DE RECHERCHE EUROPEENNE**

| | **DOCUMENTS CONSIDERES COMME PERTINENTS** | | Page  2 | |
|---|---|---|---|---|
| Catégorie | Citation du document avec indication, en cas de besoin, des parties pertinentes | Revendication concernée | CLASSEMENT DE LA DEMANDE (Int. Cl. ³) | |
| A | * En particulier figure 2; colonne 6, ligne 38 * | 9,10 | | |
| | --- | | | |
| A | US-A-3 441 136 (MILTON)<br>* En entier; en particulier colonne 6, lignes 68-71 * | 12 | | |
| | --- | | | |
| A | BE-A- 832 648 (R. BEELEN)<br><br>* Figure 1, signe de référence 6 * | | | |
| | --- | | | |
| D,A | US-A-3 920 030 (MASON)<br><br>----- | | | |
| | | | **DOMAINES TECHNIQUES RECHERCHES (Int. Cl. ³)** | |

Le présent rapport de recherche a été établi pour toutes les revendications

| Lieu de la recherche<br>LA HAYE | Date d'achèvement de la recherche<br>12-08-1982 | Examinateur<br>PETZUCH M. |
|---|---|---|

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82